# EUROPEAN PATENT APPLICATION

(11) **EP 3 998 058 A1**
(43) Date of publication of application: **18.05.2022**
(21) Application number: 20836037.0
(22) Date of filing: 08.07.2020
(51) Int. Cl.: A61K 8/41, A61K 8/67, A61K 8/9789, A61Q 19/00, A61Q 19/02, A61Q 19/08

(54) **COSMETIC COMPOSITION COMPRISING 5-BENZYLAMINOSALICYLIC ACID DERIVATIVE AND LOCAL ADMINISTRATION METHOD THEREOF**

(30) Priority: 08.07.2019 KR 20190081887
(71) Applicant: GNT Pharma Co., Ltd, Yongin-si, Gyeonggi-do 17096 (KR)
(72) Inventor: SHIN, Jin Hee, Seoul 06603 (KR); GWAG, Byoung Joo, Yongin-si, Gyeonggi-do 16948 (KR); WON, Soon Mi, Hwaseong-si, Gyeonggi-do 18505 (KR)
(74) Representative: Hübner, Gerd
(86) International application number: PCT/KR2020/008962
(87) International publication number: WO 2021/006637

(57) **Abstract**

The present invention relates to a composition comprising: a 5-benzyl aminosalicylic acid compound in chemical formula I or cosmetically acceptable salts thereof; and a cosmetically acceptable excipient suitable for local administration. The composition comprising the 5-benzyl aminosalicylic acid compound in chemical formula I or cosmetically acceptable salts thereof can be used for reducing or alleviating human skin aging by means of antioxidant, anti-inflammatory and whitening actions on the skin.

## Description

### Technical Field

The present invention relates to a cosmetic composition with anti-inflammatory, antioxidant, skin whitening and anti-aging effects, which includes 5-benzylaminosalicylic acid derivatives or cosmetically acceptable salts thereof.

The present invention provides a cosmetic composition including: a 5-benzylaminosalicylic acid derivative or a cosmetically acceptable salt thereof; and a cosmetically acceptable excipient suitable for topical administration to the skin, so as to prevent and reduce oxidative stress and inflammation, which are major risk factors for skin aging and skin damage, when applied to the skin.

The present invention provides a cosmetic composition including: 5-benzylaminosalicylic acid derivative or a cosmetically acceptable salt thereof; and a cosmetically acceptable excipient suitable for topical administration to the skin, so as to improve skin aging phenomena including skin roughness, wrinkles, pigmentation and dryness, when applied to the skin.

### Background Art

Skin aging and damage are caused by the complex action of endogenous (genetic, cell metabolism, hormones, metabolism) and exogenous (light exposure, contamination, ionization, radiation, chemical substances and toxins) factors, and are proceeding through various signaling pathways in the skin tissue. These factors cause structural and physiological variations and degenerative changes accumulated in each skin layer as well as the appearance of the skin, hence contributing to skin aging and damage. As skin aging progresses, wrinkles, roughness, dryness, and pigmented lesions occur as major features.

Oxidative stress induced by reactive oxygen species (ROS) plays an important role in endogenous and exogenous skin aging and damage processes. Endogenous aging appears as skin cell activity gradually decreases with age, and occurs by reactive oxygen species that are excessively generated during metabolic processes within skin cells. Exogenous aging occurs by external environmental factors such as photoaging due to UV, pollution and microorganisms. In particular, it is known that skin aging and damage are caused by secondary reactions via reactive oxygen species appearing when ultraviolet rays are absorbed into the skin. In addition, reactive oxygen species reduce synthesis of collagen, which is a component of connective tissues, by matrix metalloproteases (MMPs), and sometimes accelerate aging by attacking skin fibers.

Antioxidants have long been used in anti-aging cosmetics. Currently, tocopherol and its derivatives as antioxidant components are used in 86% of 287 products on the market, and vitamin C derivatives are used in 30% of products. That is, since certain antioxidants are used in a limited amount as an antioxidant component, it is needed to develop novel antioxidants with proven antioxidant activity and stability. Although alpha-tocopherol acetate (vitamin E acetate), which has higher stability than alpha-tocopherol, was used as a cosmetic ingredient, it was found a problem that skin allergies or rashes may occur and effects on the skin is uncertain. Anti-aging effects of vitamin C (ascorbate) have been well proven, but it is difficult to use vitamin C as a cosmetic ingredient due to low skin penetration and low stability. Vitamin C derivatives have been developed and used as cosmetic ingredients, but there are few results that these components are absorbed into the skin tissue to thus express effects. Therefore, it could be understood that, in order to delay skin aging and maintain normal cellular functions, discovering antioxidants with stability and safety is important.

Prostaglandin E2 (PGE₂), an inflammatory mediator, plays an important role in skin damage related to endogenous and exogenous aging factors. The expression of COX-2 (cyclooxygenase-2) as an enzyme required for PGE₂ synthesis increases in fibroblasts and keratinocytes with endogenous and exogenous aging. Treatment of cultured fibroblasts with PGE₂ reduces an amount of procollagen by 60%, and treatment with a COX-2 inhibitor reduces PGE₂ while increasing the amount of procollagen up to twice. Further, since PGE₂ stimulates melanocyte dendrite, which is involved in the movement of melanosome to keratinocytes in the skin, inhibiting COX-2 may prevent pigmentation in keratinocytes stimulated by UV.

As representative COX-2 inhibitors, salicylate and derivatives thereof, for example, butyloctyl salicylate, capryloyl salicylic acid, isocetyl salicylate, magnesium salicylate, sodium salicylate, TEA-salicylate, tridecyl salicylate, amyl salicylate, hexyl salicylate, isotridecyl salicylate, etc. have been reported to be safe for use in cosmetics at specific concentrations. However, when these COX-2 inhibitors are used topically, side effects such as skin dryness, erythema, irritation, paresthesia, itchiness, and gastrointestinal disorders have been reported, and their use as cosmetic ingredients is limited.

### Disclosure

### Technical Problem

Microsome prostaglandin E synthases 1 (mPGES-1) is a final enzyme that is directly involved in the production of PGE₂ in PGH2 (Prostaglandin H2), and it is reported that inhibitors of the above enzyme do not cause side effects such as gastrointestinal or cardiovascular damage unlike conventional COX-2 inhibitors. The expression of mPGES-1 is increased in 70% of skin tissues exposed to UV irritation and elderly skin. Therefore, it is expected that inhibiting the activity of mPGES-1 locally in the skin tissue may safely achieve anti-inflammatory and anti-aging effects.

Accordingly, the present inventors have tried to produce a cosmetic composition that can be safely used on human skin and has anti-aging effects on the skin by adopting a compound having antioxidant activity and mPGES-1 inhibitory activity.

An object of the present invention is to provide a substance that alleviates skin irritation caused by aging, various stresses or pollution, and has antioxidant, anti-inflammatory, whitening and anti-aging effects on the skin.

Another object of the present invention is to provide a cosmetic composition capable of improving skin troubles while having wrinkle improvement and whitening effects according to use of an active ingredient that removes free radicals and inhibits microsome mPGES-1 activity.

Another object of the present invention is to provide a cosmetic composition for preventing and reducing oxidative stress and inflammation that occur in the aging process of human skin.

In addition, a further object of the present invention is to provide a method of using cosmetics for preventing and reducing human skin aging.

### Technical Solution

The present invention provides a cosmetic composition with anti-inflammatory, antioxidant, skin whitening and anti-aging effects, which includes a 5-benzylaminosalicylic acid derivative represented by Formula I below or a cosmetically acceptable salt thereof.

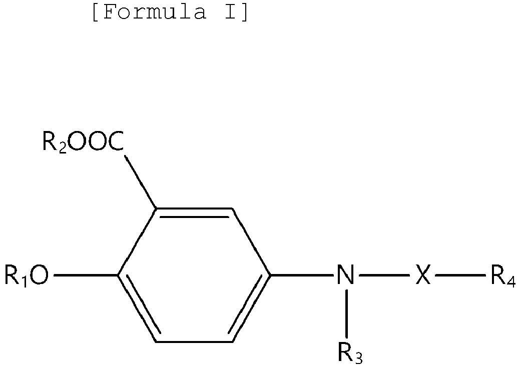

In the above formula,
X is CO, SO₂ or (CH₂)ₙ,
R₁ is hydrogen or C₁-C₆ alkyl,
R₂ is hydrogen or C₁-C₆ alkyl,
R₃ is hydrogen or C₁-C₅ acyl,
R₄ is phenyl, phenoxy, C₆-C₁₀ aryl or C₅-C₁₀ heteroaryl, wherein the aryl or heteroaryl may be substituted with one or more substituents selected from nitro, halogen, C₁-C₆ alkyl, C₁-C₆ haloalkyl, C₁-C₅ alkoxy and C₁-C₅ haloalkoxy groups, and n is an integer from 1 to 5.

The present invention provides a cosmetic composition which includes a 5-benzylaminosalicylic acid derivative represented by Formula I or a cosmetically acceptable salt thereof, so as to reduce oxidative stress and inflammation occurring in human skin aging.

The present invention provides a use of the cosmetic composition for improving human skin aging, which includes a 5-benzylaminosalicylic acid derivative represented by Formula I or a cosmetically acceptable salt thereof.

### Advantageous effects

According to the present invention, a composition including a 5-benzylaminosalicylic acid derivative represented by Formula I or a cosmetically acceptable salt thereof has effects of reducing oxidative stress and inflammation occurring in the aging process of human skin. When the composition of the present invention is used topically, it has anti-inflammatory and antioxidant effects on skin tissues, exhibits whitening effects, and attains improvement of aging such as decrease in wrinkles.

### Description of Drawings

FIG. 1 illustrates a result of confirming the particles of a nanoemulsified composition through a low-temperature scanning electron microscope (Cryo-SEM).
FIG. 2 illustrates a result of measuring a size of the nanoemulsified composition through ELS (electrophoretic light scanning) three times.
FIG. 3 illustrates a result of confirming skin brightness improvement effects in clinical practice.
FIG. 4 illustrates a result of confirming skin melanin improvement effects in clinical practice.

### Best Mode

Hereinafter, the present invention provides a cosmetic composition with anti-inflammatory, antioxidant, skin whitening and anti-aging effects, which includes a 5-benzylaminosalicylic acid derivative represented by Formula I below or a cosmetically acceptable salt thereof.

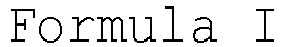

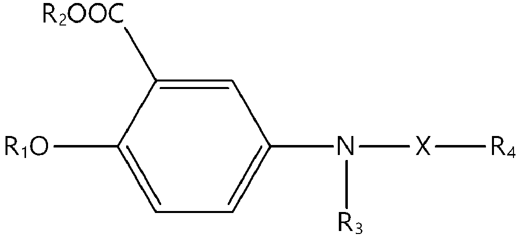

In the above formula,
X is CO, SO₂ or (CH₂)ₙ,
R₁ is hydrogen or C₁-C₆ alkyl,
R₂ is hydrogen or C₁-C₆ alkyl,
R₃ is hydrogen or C₁-C₅ acyl,
R₄ is phenyl, phenoxy, C₆-C₁₀ aryl or C₅-C₁₀ heteroaryl, wherein the aryl or heteroaryl may be substituted with one or more substituents selected from nitro, halogen, C₁-C₆ alkyl, C₁-C₆ haloalkyl, C₁-C₅ alkoxy and C₁-C₅ haloalkoxy groups, and n is an integer from 1 to 5.

The present invention provides a cosmetic composition, which includes a 5-benzylaminosalicylic acid derivative of formula I or a cosmetically acceptable salt thereof, so as to reduce oxidative stress and inflammation occurring in human skin aging.

The present inventors have prepared a cosmetic composition that could be safely used on human skin by adopting a 5-benzylaminosalicylic acid derivative having antioxidant activity and mPGES-1 inhibitory activity, and verified whether the 5-benzylaminosalicylic acid derivative has anti-aging effects on human skin, thereby completing the present invention.

The composition of the present invention may be composed of the 5-benzylaminosalicylic acid derivative of formula I or a cosmetically acceptable salt thereof.

In one embodiment, X in the compound of Formula I may be (CH₂)ₙ.

In one embodiment, with regard to the compound of Formula I, R4 may be unsubstituted phenyl, or phenyl substituted with one or more substituents selected from nitro, halogen, C₁-C₆ alkyl, C₁-C₆ haloalkyl, C₁-C₅ alkoxy and C₁-C₅ haloalkoxy groups.

In one embodiment, with regard to the compound of Formula I, R4 may be C₆-C₁₀ aryl possibly substituted with one or more substituents selected from nitro, halogen, C₁-C₆ alkyl, C₁-C₆ haloalkyl, C₁-C₅ alkoxy and C₁-C₅ haloalkoxy groups.

Preferred examples of the 5-benzylaminosalicylic acid derivative may include 5-benzylaminosalicylic acid, 2-hydroxy-5-phenethylaminobenzoic acid (compound 1), 2-hydroxy-5-[2-(4-trifluoromethyl)-phenyl)-ethylamino]-benzoic acid (compound 2), 2-hydroxy-5-[2-(3-trifluoromethyl-phenyl)-ethylamino]-benzoic acid (compound 3), 5-[2-(3,5-bis-trifluoromethyl-phenyl)-ethylamino]-2-hydroxy-benzoic acid (compound 4), 2-hydroxy-5-[2-(2-nitro-phenyl)-ethylamino]-benzoic acid (compound 5), 5-[2-(4-chloro-phenyl)-ethylamino]-2-hydroxy-benzoic acid (compound 6), 5-[2-(3,4-difluoro-phenyl)-ethylamino]-2-hydroxy-benzoic acid (compound 7), 5-[2-(3,4-dichloro-phenyl)-ethylamino]-2-hydroxy-benzoic acid (compound 8), 5-[2-(4-fluoro-2-trifluoromethyl-phenyl)-ethylamino]-2-hydroxy-benzoic acid (compound 9), 5-[2-(2-fluoro-4-trifluoromethyl-phenyl)-ethylamino]-2-hydroxy-benzoic acid (compound 10), 2-hydroxy-5-[2-(4-methoxy-phenyl)-ethylamino]-benzoic acid (compound 11), 2-hydroxy-5-(-2-o-tolyl-ethylamino)-benzoic acid (compound 12), 2-hydroxy-5-(3-phenyl-propylamino)-benzoic acid (compound 13), 2-hydroxy-5-[3-(4-trifluoromethylphenyl)-propylamino]-benzoic acid (compound 14), 5-[3-(4-fluoro-phenyl)-propylamino]-2-hydroxy-benzoic acid (compound 16), 2-hydroxy-5-(3-p-tolyl-propylamino)-benzoic acid (Compound 17), 2-acetoxy-5-[2-(4-trifluoromethyl-phenyl)-Ethylamino]-benzoic acid (compound 18), 5-[2-(2-chlorophenyl)-ethylamino]-2-hydroxy-benzoic acid (compound 19), 5-benzylaminosalicylic acid (compound 20), 5-(4-nitrobenzyl)aminosalicylic acid (compound 21), 5-(4-chlorobenzyl)aminosalicylic acid (compound 22), 5-(4-trifluoromethylbenzyl)aminosalicylic acid (compound 23), 5-(4-fluorobenzyl)aminosalicylic acid (compound 24), 5-(4-methoxybenzyl)aminosalicylic acid (compound 25), 5-(4-pentafluorobenzyl)aminosalicylic acid (compound 26), 5-(4-nitrobenzyl)amino-2-hydroxyethyl benzoate (compound 27), 5-(4-nitrobenzyl)-N-acetylamino-2-hydroxyethyl benzoate (compound 28), 5-(4-nitrobenzyl)-N-acetylamino-2-hydroxyethyl benzoate (compound 29), 5-(4-nitrobenzyl)aminosalicylic acid (compound 30), 5-(4-nitrobenzenesulfonyl)aminosalicylic acid (compound 31), 5-[2 -(4-nitrophenyl)-ethyl]aminosalicylic acid (compound 32), and 5-[3-(4-nitro-phenyl)-n-propyl]aminosalicylic acid (compound 33), but are limited thereto.

In specific and preferred embodiments, the compound of formula I is 2-hydroxy-5-[2-(4-trifluoromethylphenyl)ethylamino]benzoic acid (TFM, compound 2) or a cosmetically acceptable salt thereof.

In one embodiment, the compound of Formula I may be a compound of Formula II below.

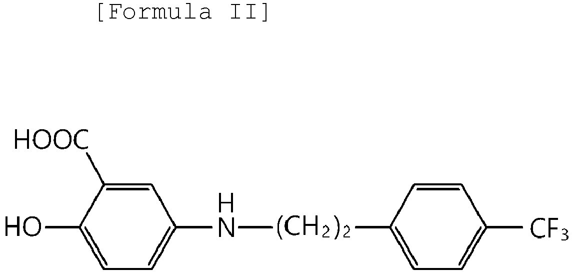

The 5-benzylaminosalicylic acid derivative of the present invention or a cosmetically acceptable salt thereof may be prepared by various known methods, including the reaction scheme described in US Pat. No. 6,573,402.

Definitions of terms described below are applicable to the use of the terms or to combinations with other terms.

An "alkyl" (including "alkyl" of haloalkyl) or "alkane" group is a fully saturated linear or branched non-aromatic hydrocarbon. Typically, linear or branched alkyl groups have 1 to about 20 carbon atoms, preferably 1 to about 10 carbon atoms, unless otherwise defined. C1-C6 linear or branched alkyl groups are also referred to as "lower alkyl" groups. In one embodiment, alkyl is C1-C6 alkyl, more preferably C1-C3 alkyl. More specifically, preferred alkyl groups may include, but are not limited to, methyl, ethyl, propyl, isopropyl, butyl, sec-butyl and tert-butyl.

Further, the term "alkyl" (or "lower alkyl") as used throughout the specification, examples and claims is intended to include both "unsubstituted alkyl" and "substituted alkyl", and the "substituted alkyl" has a substituent in which hydrogen on one or more carbons of the alkyl moiety hydrocarbon backbone is substituted. Such substituents may include, for example, halogen, hydroxyl, carbonyl (carboxyl, alkoxycarbonyl, formyl or acyl such as alkylC(0)), thiocarbonyl (e.g., thioester, thioacetate or thioformate), alkoxy, phosphoryl, phosphate, phosphonate, phostinate, amino, amido, amidine, imine, cyano, nitro, azido, silyl ether, sulfhydryl, alkylthio, sulfate, sulfonate , sulfamoyl, sulfonamino, sulfonyl, heterocyclyl, aralkyl or aromatic or heteroaromatic moieties, unless stated otherwise.

If appropriated, it will be appreciated by those skilled in the art that a substituted moiety in the hydrocarbon chain may have a substituent *per se.* For example, substituted alkyl may include, for example, a substituent such as ether, alkylthiol, carbonyl (including ketone, aldehyde, carboxylate and ester), -CF3, -CN, amino, azido, imido, amido, phosphoryl (including phosphonate and phosphinate), sulfonyls (including sulfates, sulfonamidos, sulfamoyls and sulfonates), or substituted and unsubstituted forms of silyl groups. As an example, substituted alkyl is described below. Cycloalkyl may be further substituted with alkyl, alkenyl, alkoxy, alkylthio, aminoalkyl, carbonyl-substituted alkyl, -CF3 and -CN, and the like.

The term "Cx-Cy" when used with a chemical moiety such as acyl, acyloxy, alkyl, alkenyl, alkynyl or alkoxy means that a group containing x to y carbons exists in a chain. For example, the term "Cx-Cy alkyl" refers to a substituted or unsubstituted saturated hydrocarbon group, and specifically, means a linear or branched alkyl group containing x to y carbons in a chain that contains a haloalkyl group such as trifluoromethyl and 2,2,2-trifluoroethyl. Co alkyl represents hydrogen when the group is at the terminal, or a bond when the group exist in the chain. The terms "C₂-alkenyl" and "C₂-alkynyl" refer to substituted or unsubstituted unsaturated aliphatic groups whose length and abundance of substituents are similar to the alkyls described above, but each containing one or more double or triple bonds.

When used with a chemical moiety such as acyl, acyloxy, alkyl, alkenyl, alkynyl or alkoxy, the term "lower" means to inclusion of groups having no more than 10 non-hydrogen atoms in the substituent. It is preferably 6 or less. For example, "lower alkyl" represents an alkyl group containing up to 10 carbon atoms, preferably up to 6 carbon atoms. In specific embodiments, the acyl, acyloxy, alkyl, alkenyl, alkynyl or alkoxy substituents as defined herein may be present alone or along with other substituents, for example, whether present with hydroxyalkyl and aralkyl (in this case, atoms in the aryl group are not counted when counting atoms in the alkyl substituent), and may be lower acyl, lower acyloxy, lower alkyl, lower alkenyl, lower alkynyl or lower alkoxy.

The term "alkanoyl" or "acyl" means HC(O) or hydrocarbyl-C(O)-, wherein the hydrocarbyl-C(0)- is preferably a group represented by alkyl-C(O)-. Preferably, alkanoyl is a C₂-C₁₀ alkanoyl, more preferably a C₃-C₆ alkanoyl. More specifically, preferred alkanoyls may include, but are not limited to, ethanoyl, propanoyl and cyclohexanecarbonyl. The term "alkoxy" (including "alkoxy" of haloalkoxy) refers to an alkyl group to which oxygen is attached, preferably a lower alkyl group. In one embodiment, alkoxy is preferably C₁-C₅ alkoxy, more preferably C₁-C₃ alkoxy. More specifically, preferred alkoxy may include, but is not limited to, methoxy, ethoxy and propanooxy.

The terms "amine" and "amino" refer to unsubstituted and substituted amines and salts thereof known in the art. For example, it may include an amine group represented by Formula III or Formula IV.

[Formula III] -N(R₅)₂

[Formula IV] -N(R₅)₃^{*}

R₅ represents each independently hydrogen or hydrocarbyl group, or two R₅ together form a heterocycle having 4 to 8 atoms in a ring structure along with N atom to which they are attached.

As used herein, the term "aryl" may include substituted or unsubstituted aromatic groups wherein each atom of a ring is carbon. Preferably, the ring is a 6- to 10-membered ring, more preferably a 6-membered ring.

The term "heteroaryl" as used herein may include substituted or unsubstituted heteroaromatic groups wherein one or more heteroatoms selected from N, O and S are contained in a ring. Preferably the ring is a 5- to 10-membered ring.

The term "aryl" or "heteroaryl" may include a polycyclic group having two or more rings wherein two or more carbons are common between two adjacent rings. At this time, at least one of the rings may be aromatic, and the other cyclic rings may not be aromatic, for example, cycloalkyl, cycloalkenyl, aryl, heteroaryl and/or heterocyclyl.

The aryl or heteroaryl of the present invention includes benzene, naphthalene, phenanthrene, phenol, aniline, and the like. Exemplary substituents for the aryl group may include, for example, halogen, haloalkyl such as trifluoromethyl, hydroxyl, carbonyl (carboxy, alkoxycarbonyl, formyl or acyl such as alkyl C(0)), alkoxy phosphoryl, phosphonate, phosphinate, amino, amidine, cyano, nitro, azido, silyl ether, sulfhydryl, alkylthio, sulfate, sulfonate, sulfamoyl, sulfonamido, sulfonyl, heterocyclyl, aralkyl, aromatic moieties or heteroaromatic moieties.

As used herein, the terms "halo" and "halogen" mean halogen and may include chloro, fluoro, bromo and iodine.

The term "substituted" means to having a substituent to substitute hydrogen on one or more carbons of the main chain. It will be understood that "substitution" or "substituted" includes such implicit conditions that the substitution defers to the substituted atom and the allowed valence of the substituent, and that a stable compound not occurring spontaneously is produced, for example, by the substitution. The term "substituted" used herein is contemplated to include all acceptable substituents of an organic compound. In a broad aspect, acceptable substituents may include bicyclic and cyclic, branched and linear chain, carbocyclic and heterocyclic, aromatic and non-aromatic substituents of organic compounds. One or more acceptable substituents may exist for a suitable organic compound and, if two or more, these substituents may be the same or different. For the purposes of the present invention, heteroatoms such as nitrogen may be used for hydrogen substituents and/or any substituent described herein that satisfy the valence of the heteroatom, e.g. halogen, haloalkyl, hydroxyl, carbonyl (carboxy, alkoxy Carbonyl, formyl or acyl), thiocarbonyl (thioester, thioacetate), phosphonate, phosphinate, amino, amido, amidine, imine, cyano, nitro, azido, sulfhydryl, alkylthio, sulfate, sulfonate, sulfamoyl, sulfonamido, sulfonyl, heterocyclyl, aralkyl, or aromatic or heteroaromatic moieties. If appropriate, it will be understood by those skilled in the art that the substituents may also be substituted by themselves. Unless specifically stated as "unsubstituted", a chemical moiety mentioned herein would be understood to include substituted variants. For example, "aryl" groups or moieties may implicitly include both substituted and unsubstituted variants.

The term "cosmetically acceptable salt" of the present invention refers to a salt produced with a non-toxic or almost non-toxic acid or base. When the compound of the present invention is acidic, the base addition salt of the compound according to the present invention may be prepared by reacting free base of the compound with a sufficient amount of desirable base and an appropriate inert solvent. Cosmetically acceptable base addition salts may include, but are not limited to, salts prepared with sodium, potassium, calcium, ammonium, magnesium or organic amino.

When a compound of the present invention is basic, the free base of the compound may react with a sufficient amount of desirable acid and a suitable inert solvent, in order to prepare an acid addition salt of the compound. Cosmetically acceptable acid addition salts may include salts of propionic acid, isobutyric acid, oxalic acid, malic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, hydrochloric acid, bromic acid, nitric acid, carbonic acid, monohydrocarbonic acid, phosphoric acid, monohydrogen phosphoric acid, sulfuric acid, monohydrogen sulfuric acid, hydrogen iodide or phosphorous acid. Further, cosmetically acceptable salts of the present invention may include, but are not limited to, salts of amino acids such as alginate and analogs of organic acids such as gluculonic acid or galacturonic acid.

For example, a cosmetically acceptable salt of 2-hydroxy-5-[2-(4-trifluoromethylphenyl)-ethylamino]-benzoic acid (compound 2) which is a preferred example of the present disclosure may be prepared by the following reaction method, but the scope of the present invention is not limited thereto.

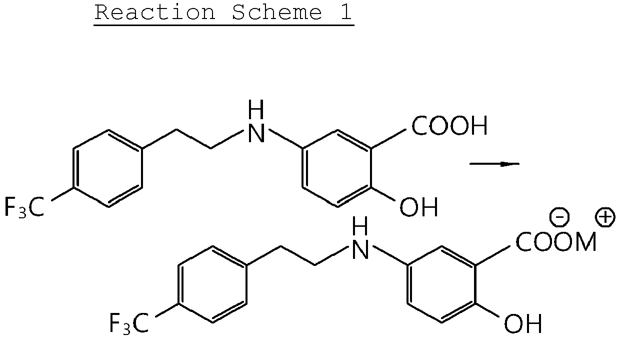

In the above scheme, M is a cosmetically acceptable metal or basic organic compound, such as diethylamine, lithium, sodium or potassium.

More specifically, the diethylamine salt may be produced by dissolving the compound in alcohol, adding diethylamine dropwise, stirring and distilling the mixture under vacuum, and adding ether in order to crystallize the residue. The alkali metal salt may be produced by preparing a preferred salt with an inorganic reagent such as lithium hydroxide, sodium hydroxide or potassium hydroxide in a solvent such as alcohol, acetone or acetonitrile, and then freeze-drying the same. Further, the lithium salt may be prepared from lithium acetate, the sodium salt may be prepared from sodium 2-ethylhexanite or sodium acetate, and the potassium salt may be prepared from potassium acetate.

Some of the compounds according to the present disclosure may be in hydrated form, and may exist in solvated or unsolvated forms. Some of the compounds according to the invention may exist in crystalline or amorphous form and are included in any physical form in the scope of the invention. In addition, some compounds of the present invention may contain one or more asymmetric carbon atoms or double bonds, thus existing in two or more isomeric forms such as racemates, enantiomers, diastereomers, geometric isomers, and the like. The present invention may include individual isomers of these compounds.

### Composition

The present invention also provides a composition that includes a 5-benzylaminosalicylic acid derivative represented by Formula I above or a cosmetically acceptable salt thereof, as well as a cosmetically acceptable excipient or additive. The 5-benzylaminosalicylic acid derivative represented by Formula I or a cosmetically acceptable salt thereof may be administered alone. In one embodiment, the composition including the compound represented by Formula I may include any suitable excipients, diluents, and the like.

The excipients or additives may be used without limitation as far as the excipients or additives are used in the manufacture of cosmetic compositions, and preferably include ceramide, nonionic surfactant, higher alcohol, cholesterol, ester oil, propylene glycol, beta-sitosterol, vegetable oils, etc.

In one embodiment, the cosmetic composition of the present invention may include a compound of Formula I or a cosmetically acceptable salt thereof in an amount of 0.001 to 1 % by weight ("wt.%"), preferably 0.01 to 0.1 wt.%, more preferably 0.02 to 0.1 wt.% to a total weight of the composition. When included as described above, antioxidant, skin soothing enhancement, anti-inflammatory, whitening or wrinkle improvement effects may be obtained.

In one embodiment, the cosmetic composition of the present invention may be a nano-emulsifying cosmetic composition that includes a 5-benzylaminosalicylic acid derivative or a cosmetically acceptable salt thereof, which is emulsified in a nano size in order to increase skin absorption and obtain higher effects on the skin.

The nano-emulsifying cosmetic composition may further include glycerin, triethanolamine, trisodium IDTA, dipropylene glycol, hydrogenated lecithin, vitamins, peptides, etc. as an aqueous-phase component, and vegetable squalane, phenyltrimethicone, cyclopentasiloxane, cetyloctanoate, meadow foam seed oil, dimethicone, tocopheryl acetate, beeswax, etc. as an oil-phase component, in addition to the 5-benzylaminosalicylic acid derivative.

The nano-emulsifying cosmetic composition may include 0.02 to 0.1 wt.% of the compound of Formula I, 30 to 60 wt.% of lecithin and glycerin, 5 to 20 wt.% of polyglyceryl-10 oleate, 1 to 5 wt.% of 1,2-hexanediol, and 1 to 10 wt.% of hydrogenated lecithin.

A method for production of the nano-emulsion composition according to the present invention will be described below, but is not limited thereto.

In order to commercialize the compound of Formula I as a cosmetic product, the present invention first prepares an aqueous solution of the compound of Formula I in an amount of 0.02 to 0.1 wt.% to a total weight of the final cosmetic composition, as well as an aqueous-phase component mixture in which 2 to 10 wt.% of hydrogenated lecithin and 5 to 15 wt.% of pollyglycerin are contained as emulsifiers in a separate dissolution bath. Thereafter, the aqueous solution of the compound of Formula I may be added to the aqueous-phase component mixture containing lecithin and polyglycerin, followed by premixing the mixture using a homomixer or a paddle mixer, thus obtaining a first pre-emulsified composition. Then, the pre-emulsified composition may be put in a microfluidizer and secondly emulsified three times at 1000 bar to prepare a nanoemulsified cosmetic composition containing the compound of Formula I.

Lecithin used as an emulsifier in the present invention is a substance also called phosphatidyl choline as one of the major phospholipid components constituting the cell membrane system of animals, and has a structure in which hydrophilic components such as phosphoric acid and choline are bound to one side of glycerol and a hydrophobic acyl group is bonded to the other side, thereby exhibiting surface activity. Such lecithin has a constitutional composition similar to that of a lipid component in the skin and shows higher skin affinity, whereby skin permeability of the stratum corneum may be promoted during transdermal absorption of cosmetics.

The nanoemulsified cosmetic composition of the present invention may form fine and uniform particles having an average particle size of 100-150 nM. Therefore, when a cosmetic product including the nanoemulsified cosmetic composition of the present invention is manufactured, a cosmetic with excellent formulation quality, significantly improved transdermal absorption rate of an active ingredient, in turn, great wrinkle improvement and whitening effects may be produced.

Glycerin, polyglyceryl-10 oleate, 1,2-hexanediol and hydrogenated lecithin are used as the aqueous-phase components of the nanoemulsified cosmetic composition containing the compound of formula II.

### Functional cosmetics

The present invention provides a functional cosmetic product including the above composition. The cosmetics of the present invention may be commercialized as a cosmetic product and used topically on the skin. The cosmetic product of the present invention may be in the form of ampoules, masks, creams, serums, balms or gels for the face or body.

The functional cosmetics of the present invention may be used for antioxidation, skin soothing enhancement, anti-inflammation, whitening and/or wrinkle improvement. In order attain the above effects, the functional cosmetics of the present invention may contain 0.0001 to 0.1 wt.% of the compound of formula I as an active ingredient.

For wrinkle improvement, the cosmetics of the present invention may include at least one selected from retinol, retinyl palmitate, adenosine and polyethoxylated retinamide, in addition to the 5-benzylaminosalicylic acid derivative of Formula I or a cosmetically acceptable salt thereof.

For whitening, the cosmetics of the present invention may include at least one selected from oak tree extract, arbutin, ethylascorbyl ether, oil-soluble licorice extract, ascorbyl glucoside, magnesium ascorbyl phosphate, niacinamide, alpha-bisabolol and ascorbyl tetraisopalmitate.

The functional cosmetics of the present invention may be used in any one of forms among pastes, gels, creams, lotions, powders, solid soaps, water soaps, shampoos, rinses, solutions, suspensions, emulsions, mineral cosmetics, powder perfumes, surfactant-containing cleansings or surfactant-free cleansings, oil, powder foundation, emulsion foundation, hair dye, wax, skin lotion, nutrition lotion, nutrition cream, massage cream, essence, cleansing cream, cleansing foam, pack, base, eye cream, fragrance, ointment, cleansing water, wax foundation and spray.

The present invention also provides the use of a 5-benzylaminosalicylic acid derivative or a cosmetically acceptable salt thereof for preventing and reducing oxidative stress and inflammation during the aging process of human skin. In other words, the present invention provides a cosmetic composition for improving human skin aging or cosmetics including the same, each of which includes the 5-benzylaminosalicylic acid derivative represented by Formula I or a cosmetically acceptable salt thereof. More specifically, a cosmetic composition or cosmetics including the same, each of which includes a 5-benzylaminosalicylic acid derivative or a cosmetically acceptable salt thereof, may be used to improve human skin aging, including skin texture, wrinkles, coloring and drying.

In one embodiment, the composition of the present invention may be used in the manufacture of cosmetics for improving the skin aging process in humans. In one embodiment, the composition of the present invention may be used in the manufacture of cosmetics for improving the skin aging process through pharmacological inhibition of oxidative stress and inflammation. In one embodiment, the composition of the present invention may be used in the manufacture of cosmetics for improving the skin aging process by inhibiting oxidative stress and prostaglandin E₂ synthesis. In one embodiment, the composition of the present invention may be used in the manufacture of cosmetics for skin improvement by inhibiting oxidative stress and microsomal prostaglandin E synthase-1.

### Detailed Description of Preferred Embodiments of Invention

Hereinafter, it will be described in detail so that those skilled in the art may understand the present disclosure by way of an exemplary embodiment. However, the following examples are provided for illustrative purposes and are not intended to limit the scope of the present invention. Briefly, it would be obviously understood that various changes are possible without departing from the spirit and scope of the present invention or without sacrificing all material advantages.

### Example

### Example 1. Safety assessment for human skin

Safety of Compound 2 was assessed by a patch test of 33 human volunteers. At this time, a patch treated with a composition containing 0.0544 mg/ml of compound 2 was attached to the test site, after 24 hours, the patch was removed and the location was marked. After 1 hour, a degree of reactivity was determined and recorded. Further, after 24 hours, the degree of reactivity and a skin irritation index were evaluated and shown in Tables 2 and 3 below. The criteria for determining a degree of skin reaction are shown in Table 1 below.

**TABLE 1**

| **sign** | **Grade** | **Criteria** |
|---|---|---|
| + | 1 | Faint erythema |
| ++ | 2 | Erythema |
| +++ | 3 | Severe erythema with edema |
| ++++ | 4 | Severe erythema with edema and vesicles |

The skin reactivity was calculated by multiplying the average of evaluated values for 33 subjects by 25, while the skin irritation index was determined by dividing the above calculated value by the number of evaluations.

The degree of skin irritation of the test substance was determined with reference to the skin irritation index table (Table 1).

**TABLE 2**

| **Skin irritation index** | **Division** |
|---|---|
| 0.00-0.25 | Non-irritation |
| 0.26-1.00 | Weak irritation |
| 1.01-2.50 | Moderate irritation |
| 2.51-4.00 | Strong irritation |

**TABLE 3**

| | **Skin Irritation Index** | **Degree of Skin Irritation** |
|---|---|---|
| **Compound 2** | 0 00 | - |

Referring to Table 3, there were no reports of specific adverse skin reactions during the period of using compound 2, and no abnormal findings were observed even on a physical examination by a dermatologist.

Example 2. Preparation of nano-emulsified composition

In order to increase the transdermal absorption rate of compound 2, 0.05 wt.% of compound 2, 50 wt.% of glycerin, 10.00 wt.% of polyglyceryl-10 oleate, 2.00 wt.% of 1,2-hexanediol, 5.00 wt.% of hydrogenated lecithin and water were premixed with a homomixer to obtain a first pre-emulsified composition. The first composition was put in a microfluidizer, followed by secondly emulsifying the same three times at 1000 bar to prepare a nanoemulsified composition composed of fine and uniform particles with a size of 100 to 150 nM.

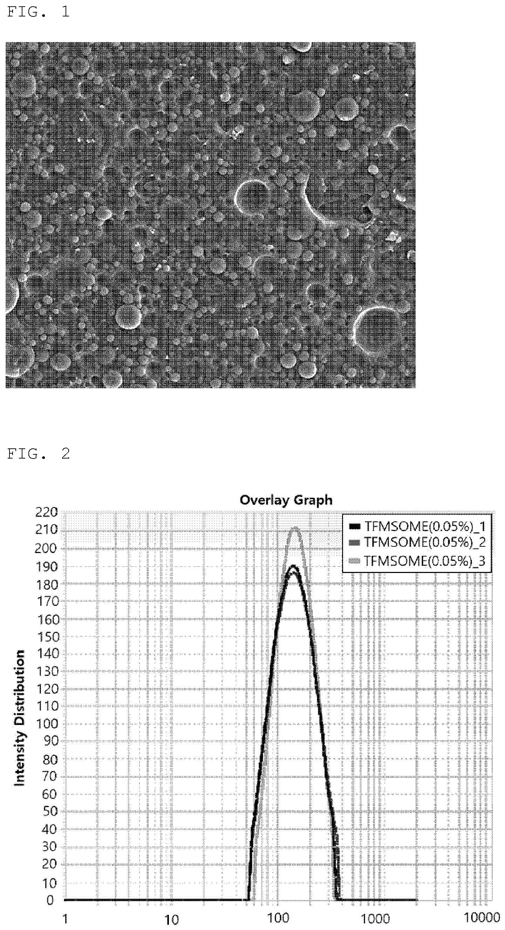

Formation of liposomes was confirmed through a low-temperature scanning microscope, and a size of the nanoemulsion composition was measured using ELS (electrophoretic light scattering). Results thereof are shown in FIGS. 1 and 2, respectively.

Example 3. Preparation of test product and control product

As a test product, 0.064 wt.% of the nanoemulsified composition of Example 2 (0.0032 wt.% of compound 2), 30 wt.% of glycerin, 28.5 wt.% of snail mucus filtrate, 5.0 wt.% of butylene glycol, 3.0 wt.% of isopentyldiol, and purified water were prepared to form an ampoule.

As a control product, a product including the above components except for the nanoemulsified composition of the present invention was prepared.

Example 4. Wrinkle improvement and whiting effects in clinical practice

In order to confirm effects of the ampoule containing compound 2 on wrinkles and whitening, an experiment was conducted on 22 healthy Korean women aged 30 to 55 years (hereinafter referred to as "subjects"). Each of the subjects was given the ampoule containing compound 2 (test product), and tested by continuously applying an appropriate amount of the content in the ampoule twice each morning and evening for 2 months such that the content is absorbed along the skin texture from the inside of the face including the wrinkles around the eyes to the outside.

Sites to be measured for the degree of wrinkles and pigmentation were the subject's eye area and cheek area. The face was divided into halves, and the left and right halves were designated as specific areas. The test product was applied on one half of the face while applying the control product on the other half. In order to determine the degree of wrinkles and pigmentation, the subject has rested for 30 minutes in a waiting room under controlled temperature and humidity condition, for example, at 20 to 25 °C under 40 to 60% internal humidity, so that the skin surface temperature and humidity were adjustable to the environment of the measurement space. At this time, water intake was limited. For objective measurement, a researcher has measured the same area before using the product (visit 1), 4 weeks (visit 2), and 8 weeks (visit 3) after using the product.

### (1) wrinkle improvement effects

With regard to the area to which the test product and the control product were applied before, 4 and 8 weeks after use, skin replicas were prepared and analyzed using a Visiometer SV700 (Courage_Khazaka electronic GmbH, Germany) and through transparency profilometry analsysis, so as to assay the effect of improving wrinkles on each subject. In this case, wrinkle parameters used herein were R1, R2, R3, R4, and R5 (R1: skin roughness, R2: maximum roughness, R3: average roughness, R4: smoothness depth, R5: arithmetic average roughness), respectively. Results of the analysis are shown in Tables 4 and 5 below.

**TABLE 4**

| | **R1 (Skin roughness)** | | **R2 (Maximum roughness)** | | **R3 (Average roughness)** | |
|---|---|---|---|---|---|---|
| | Test group | Control | Test group | Control | Test group | Control |
| Before use | 0.385±0.0 60 | 0.361±0.0 46 | 0.257±0.0 27 | 0.250±0.0 35 | 0.202±0.0 21 | 0.199±0.0 26 |
| 4 weeks later | 0.353±0.0 54 | 0.370±0.0 52 | 0.243±0.0 23 | 0.251±0.0 24 | 0.187±0.0 17 | 0.196±0.0 20 |
| Differen ce | 0.032 | -0.009 | 0.014 | -0.001 | 0.015 | 0.003 |
| P | 0.006* | 0.877 | 0.070* | / | 0.020* | / |
| 8 weeks later | 0.328:1:0.0 51 | 0.388±0.0 49 | 0.237±0.0 30 | 0.256±0.0 30 | 0.186±0.0 18 | 0.199±0.0 22 |
| Differen ce | 0.057 | -0.027 | 0.02 | -0.006 | 0.016 | 0.00 |
| *p* | 0.000* | 0.028* | 0.025* | / | 0.004* | / |

**TABLE 5**

| | **R4 (smoothness depth)** | | **R5 (Arithmetic average)** | |
|---|---|---|---|---|
| | Test group | Control | Test group | Control |
| Before use | 0.200±0.041 | 0.193±0.034 | 0.058±0.019 | 0.053±0.012 |
| 4 weeks later | 0.195±0.044 | 0.200±0.036 | 0.054±0.017 | 0.055±0.013 |
| Difference | 0.005 | -0.007 | 0.004 | -0.002 |
| P | / | 1.000 | 0.102 | 0.519 |
| 8 weeks later | 0.177±0.043 | 0.251±0.035 | 0.047±0.012 | 0.062±0.015 |
| Difference | 0.023 | -0.058 | 0.011 | -0.009 |
| P | / | 0.071 | 0.004* | 0.025 |

In the examples using the test product (ampoule containing compound 2), the skin wrinkle parameters R1, R2, R3, and R5 were significantly reduced, and it also showed a tendency to decrease R4. Therefore, it was confirmed that the skin wrinkle improvement effects are very excellent.

### (2) Whitening effects

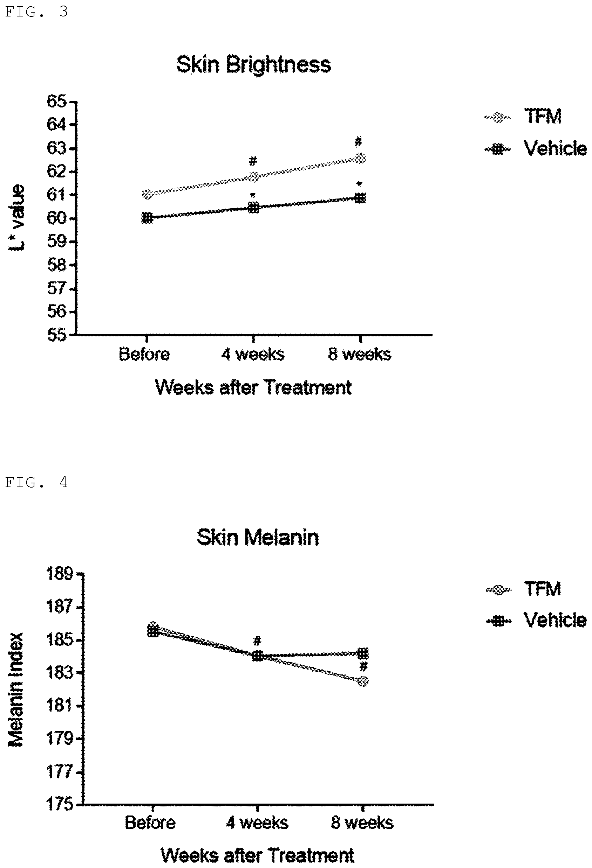

Skin brightness was measured three times using Spectrometer CM700-d (Konica Minolta, Japan), and the average of the measured values was used as skin brightness evaluation data. In this regard, the skin brightness was evaluated using L* value among the measurement variables, and skin melanin was measured by means of Mexameter MX18 (courage + Khazaka electronic GmbH, Germany). Maxameter is a narrow-band reflectance spectro-photometer that measures the light reflected from the skin by emitting light at 568 nm (green), 660 nm (red) and 880nm (infared), respectively, from ae probe, and has a unit of M.I (Melanin Index). Results of the above measurements are shown in FIGS. 3 and 4.

In the examples using the test product (ampoule containing compound 2), the L* value representing skin brightness was significantly increased while decreasing M.I value, thereby confirming that skin whitening effects are very excellent.

Hereinafter, non-limiting preparative examples of the cosmetic products of the present invention which were formulated for topical use on the skin of the body or face will be described.

### Preparative Example 1. Nanoemulsified composition

The components of the nanoemulsified composition are shown in Table 6.

**TABLE 6**

| **Ingredient** |
|---|
| Compound 2 |
| Hydrogenated Lecithin |
| Water |
| Glycerin |
| Polyglyceryl-10 Oleate |
| 1,2-Hexanediol |
| Trifluoromethyl Phenethyl Mesalazine |

### Preparative Example 2. Preparation of ampoule

An ampoule was prepared with the constitutional composition shown in Table 7 below.

**TABLE 7**

| **Ingredient** |
|---|
| Compound 2 |
| Glycerin |
| Snail Secretion Filtrate |
| Leontopodium Alpinum Callus Culture Extract |
| Butylene Glycol |
| Isopentyldiol |
| Glycereth-26 |
| Theobroma Cacao (Cocoa) Seed Extract |
| 1,2-Hexanediol |
| Caprylic/Capric Triglyceride |
| Trifluoromethylphenethyl Mesalazine |
| Hydrogenated Lecithin |
| Polyglyceryl-10 Oleate |
| Centella Asiatica Extract |
| Palmitoyl Tripeptide-5 |
| Dipeptide Diaminobutyroyl Benzylamide Diacetate |
| Hydrolyzed Hyaluronic Acid |
| Beta-Glucan |
| Sodium Polyacrylate |
| Cetearyl Olivate |
| Sorbitan Olivate |
| Ethylhexylglycerin |
| Phenyl trimethicone |
| Dimethicone/Vinyl dimethicone Crosspolymer |
| Disodium EDTA |
| Water |

### Preparative Example 3. Preparation of toner

A toner was prepared with the constitutional composition shown in Table 8 below.

**TABLE 8**

| **Ingredient** |
|---|
| Compound 2 |
| Butylene Glycol |
| Glycerin |
| Cetyl Ethylhexanonate |
| 1,2-Hexanediol |
| Squalane |
| Polyglyceryl-3 Methylglucose Distearate |
| Caprylic/Capric Triglyceride |
| Acrylate/C10-30 Alkyl Acrylate Crosspolymer |
| Cetyl Alcohol |
| Trifluoromethylphenethyl Mesalazine |
| Betaine |
| Hydrogenated Lecithin |
| Polyglyceryl-10 Oleate |
| Sodium Hyaluronate |
| Centella Asistic Extract |
| Panthenol |
| Citric Acid |
| Glycereth-26 |
| Allantoin |
| Glyceryl Stearate |
| Stearic Acid |
| Palmitic Acid |
| Sorbitan Sesquioleate |
| Vinyl Dimethicone |
| Water |

Preparative Example 4. Preparation of lotion A lotion was prepared with the constitutional composition shown in Table 9 below.

**TABLE 9**

| **Ingredient** |
|---|
| Compound 2 |
| Butylene Glycol |
| Glycerin |
| 1,2-Hexanediol |
| Methylgluceth-20 |
| Trifluoromethylphenethyl Mesalazine |
| Hydrogenated Lecithin |
| Polyglyceryl-10 Oleate |
| Sodium Hyaluronate |
| Centella Asiatica |
| Betaine |
| Glycereth-26 |
| Hydrolyzed Jojoba Esters |
| Allantoin |
| Xanthan Gum |
| Acrylates/C10-30Alkyl Acrylate Crosspolymer |
| Propanediol |
| Plyglyceryl-10 Laurate |
| Tromethamine |
| Boswellia Carterii Oil |
| Water |

### Preparative Example 5. Preparation of mask sheet

A mask sheet was prepared with the constitutional composition shown in Table 10 below.

**TABLE 10**

| **Ingredient** |
|---|
| Compound 2 |
| Glycerin |
| 1,2-hexandiol |
| Butylene Glycol |
| Sodium Hyaluronate |
| Hydrolyzed Hyaluronic Acid |
| Beta-Glucan |
| Centella Asiatica Extract |
| Dipeptide Diaminobutyroyl Benzylamide Diacetate |
| Palmitoyl Tripeptide-5 |
| Trifluoromethylphenethyl Mesalazine |
| Glycereth-26 |
| Tocopheryl Acetate |
| Allantoin |
| Squalane |
| Limnanthes Alba (Meadowfoam) Seed Oil |
| Acrylateds/C10-30 Alkyl Acrylate Crosspolymer |
| Xanthan Gum |
| Caprylic/Capric Triglyceride |
| Hydrogenated Lecithin |
| Behenyl Alcohol |
| Glyceryl Stearate |
| Ceramide NP |
| Cellulose Gum |
| Disodium EDTA |
| Water |

### Industrial Applicability

The cosmetic composition with anti-inflammatory, antioxidant, skin whitening and anti-aging effects, which includes 5-benzylaminosalicylic acid derivative or a cosmetically acceptable salt thereof according to the present invention may be used in the manufacture of cosmetics.

## Claims

1. A cosmetic composition with anti-inflammatory, antioxidant, skin whitening and anti-aging effects, comprising:
a 5-benzylaminosalicylic acid derivative represented by Formula I below or a cosmetically acceptable salt thereof/ 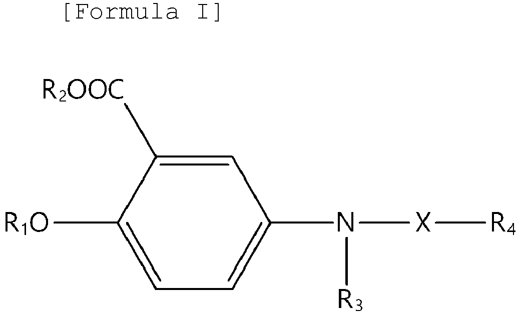 wherein, in the above formula,
X is CO, SO₂ or (CH₂)ₙ,
R₁ is hydrogen or C₁-C₆ alkyl,
R₂ is hydrogen or C₁-C₆ alkyl,
R₃ is hydrogen or C₁-C₅ acyl,
R₄ is phenyl, phenoxy, C₆-C₁₀ aryl or C₅-C₁₀ heteroaryl, wherein the aryl or heteroaryl can be substituted with one or more substituents selected from nitro, halogen, C₁-C₆ alkyl, C₁-C₆ haloalkyl, C₁-C₅ alkoxy and C₁-C₅ haloalkoxy groups, and
n is an integer from 1 to 5.

2. The cosmetic composition according to claim 1, wherein X is (CH₂)ₙ, R₄ is unsubstituted phenyl or phenyl substituted with one or more substituents selected from nitro, halogen, C₁-C₆ alkyl, C₁-C₆ haloalkyl, C₁-C₅ alkoxy and C₁-C₅ haloalkoxy groups.

3. The cosmetic composition according to claim 1, wherein the 5-benzylaminosalicylic acid derivative is any one or more selected from the group consisting of: 5-benzylaminosalicylic acid; 2-hydroxy-5-phenethylaminobenzoic acid (compound 1); 2-hydroxy-5-[2-(4-trifluoromethyl)-phenyl)-ethylamino]-benzoic acid (compound 2); 2-hydroxy-5-[2-(3-trifluoromethylphenyl)-ethylamino]-benzoic acid (compound 3); 5-[2-(3,5-bis-trifluoromethyl-phenyl)-ethylamino]-2-hydroxy-benzoic acid (compound 4); 2-hydroxy-5-[2-(2-nitro-phenyl)-ethylamino]-benzoic acid (compound 5); 5-[2-(4-chloro-phenyl)-ethylamino]-2-hydroxy-benzoic acid (compound 6); 5-[2-(3,4-difluoro-phenyl)-ethylamino]-2-hydroxy-benzoic acid (compound 7); 5-[2-(3,4-dichloro-phenyl)-ethylamino]-2-hydroxy-benzoic acid (compound 8); 5-[2-(4-fluoro-2-trifluoromethyl-phenyl)-ethylamino]-2-hydroxy-benzoic acid (compound 9); 5-[2-(2-fluoro-4-trifluoromethyl-phenyl)-ethylamino]-2-hydroxy-benzoic acid (compound 10); 2-hydroxy-5-[2-(4-methoxy-phenyl)-ethylamino]-benzoic acid (compound 11); 2-hydroxy-5-(-2-o-tolyl-ethylamino)-benzoic acid (compound 12); 2-hydroxy-5-(3-phenyl-propylamino)-benzoic acid (compound 13); 2-hydroxy-5-[3-(4-trifluoromethylphenyl)-propylamino]-benzoic acid (compound 14); 5-[3-(4-fluoro-phenyl)-propylamino]-2-hydroxy-benzoic acid (compound 16); 2-hydroxy-5-(3-p-tolyl-propylamino)-benzoic acid (Compound 17); 2-acetoxy-5-[2-(4-trifluoromethyl-phenyl)-Ethylamino]-benzoic acid (compound 18); 5-[2-(2-chlorophenyl)-ethylamino]-2-hydroxy-benzoic acid (compound 19); 5-benzylaminosalicylic acid (compound 20); 5-(4-nitrobenzyl)aminosalicylic acid (compound 21); 5-(4-chlorobenzyl)aminosalicylic acid (compound 22); 5-(4-trifluoromethylbenzyl)aminosalicylic acid (compound 23); 5-(4-fluorobenzyl)aminosalicylic acid (compound 24); 5-(4-methoxybenzyl)aminosalicylic acid (compound 25); 5-(4-pentafluorobenzyl)aminosalicylic acid (compound 26); 5-(4-nitrobenzyl)amino-2-hydroxyethyl benzoate (compound 27); 5-(4-nitrobenzyl)-N-acetylamino-2-hydroxyethyl benzoate (compound 28); 5-(4-nitrobenzyl)-N-acetylamino-2-hydroxyethyl benzoate (compound 29); 5-(4-nitrobenzyl)aminosalicylic acid (compound 30); 5-(4-nitrobenzenesulfonyl)aminosalicylic acid (compound 31); 5-[2 -(4-nitrophenyl)-ethyl]aminosalicylic acid (compound 32); and 5-[3-(4-nitro-phenyl)-n-propyl]aminosalicylic acid (compound 33) .

4. The cosmetic composition according to claim 3, wherein the compound of Formula I is 2-hydroxy-5-[2-(4-trifluoromethylphenyl)ethylamino]-benzoic acid.

5. The cosmetic composition according to claim 1, wherein the composition is a nanoemulsified composition.

6. The cosmetic composition according to claim 1, wherein the compound of Formula 1 or a cosmetically acceptable salt thereof is contained in an amount of 0.001 to 1% by weight ("wt.%") to a total weight of the composition.

7. A functional cosmetic product including the cosmetic composition according to claim 1.

8. The functional cosmetic product according to claim 7, wherein, for wrinkle improvement, the cosmetic product includes at least one selected from retinol, retinyl palmitate, adenosine and polyethoxylated retinamide.

9. The functional cosmetic product according to claim 7, wherein, for whitening, the cosmetic product includes at least one selected from oak tree extract, arbutin, ethylascorbyl ether, oil-soluble licorice extract, ascorbyl glucoside, magnesium ascorbyl phosphate, niacinamide, alpha-bisabolol and ascorbyl tetraisopalmitate.

10. The functional cosmetic product according to claim 7, wherein the cosmetic product is used for antioxidation, skin soothing enhancement, anti-inflammation, whitening or wrinkle improvement.

11. The functional cosmetic product according to claim 7, wherein the cosmetic product is used in any one of forms among pastes, gels, creams, lotions, powders, solid soaps, water soaps, shampoos, rinses, solutions, suspensions, emulsions, mineral cosmetics, powder perfumes, surfactant-containing cleansings or surfactant-free cleansings, oil, powder foundation, emulsion foundation, hair dye, wax, skin lotion, nutrition lotion, nutrition cream, massage cream, essence, cleansing cream, cleansing foam, pack, base, eye cream, fragrance, ointment, cleansing water, wax foundation and spray.
